# EUROPÄISCHE PATENTANMELDUNG

(11) **EP 1 810 685 A1**
(43) Veröffentlichungstag der Anmeldung: **25.07.2007**
(21) Anmeldenummer: 06090172.5
(22) Anmeldetag: 13.01.2003
(51) Int. Cl.: A61K 38/17, C07K 14/47, C12N 15/12, C12N 1/21, C12N 5/10

(54) **Verwendung von dimer-spezifischen Nuklear-Lokalisations-Signalpeptiden (dsNLS) abgeleitet aus der STAT DNA Bindungsdomäne**

(30) Priorität: 17.01.2002 DE 10201791
(62) Teilanmeldung aus: 03702426.2
(71) Anmelder: Forschungsverbund Berlin e.V., 60594 Frankfurt am Main (DE)
(72) Erfinder: Meyer, Thomas, 34225 Baunatal (DE); Vinkemeier, Uwe, 13189 Berlin (DE)
(74) Vertreter: Boeckh, Tobias

(57) **Zusammenfassung**

Die Erfindung betrifft die Verwendung von dimerspezifischen Nuklear-Lokalisations-Signalpeptiden (dsNLS) abgeleitet aus der STAT DNA Bindungsdomäne zur Diagnose und/oder Therapie von mit ihnen in Zusammenhang stehenden Krankheiten und als Targetmoleküle zum Screening von pharmakologischen Wirkstoffen.

SEQUENCE LISTING

<110>  Forschungsverbund Berlin e.V.

<120>  Verwendung von dimer-spezifischen
       Nuklear-Lokalisations-Signalpeptiden (dsNLS) abgeleitet aus der
       STAT DNA Bindungsdomäne

<130>  XII 796-06

<160>  8

<170>  PatentIn version 3.3

<210>  1
<211>  22
<212>  PRT
<213>  Künstliche Sequenz


<220>
<221>  MISC_FEATURE
<223>  Beschreibung der künstlichen Sequenz: Peptid

<400>  1

Leu Leu His Glu Leu Thr Ile Gln Ser Ile Gln Leu Glu Leu Tyr His
1               5                   10                  15


Glu Glu Leu Gln Leu Leu
            20


<210>  2
<211>  16
<212>  PRT
<213>  Künstliche Sequenz


<220>
<221>  MISC_FEATURE
<223>  Beschreibung der künstlichen Sequenz: Peptid

<400>  2

His Val Phe Ser Ala Ser Phe Arg Asn Met Gln Leu Lys Lys Ile Lys
1               5                   10                  15


<210>  3
<211>  16
<212>  PRT
<213>  Künstliche Sequenz


<220>
<221>  MISC_FEATURE
<223>  Beschreibung der künstlichen Sequenz: Peptid

<400>  3

Asn Cys Cys Ser Ala Leu Phe Lys Asn Leu Leu Leu Lys Lys Ile Lys
1               5                   10                  15


<210>  4
<211>  15

<212>  PRT
<213>  Künstliche Sequenz

<220>
<221>  MISC_FEATURE
<223>  Beschreibung der künstlichen Sequenz: Peptid

<400>  4

Thr Leu Ser Ala His Phe Arg Asn Met Ser Leu Lys Arg Ile Lys
1               5                   10                  15

<210>  5
<211>  19
<212>  PRT
<213>  Künstliche Sequenz

<220>
<221>  MISC_FEATURE
<223>  Beschreibung der künstlichen Sequenz: Peptid

<400>  5

Ser Leu Ser Val Glu Phe Arg His Leu Gln Pro Lys Glu Met Lys Ser
1               5                   10                  15

Ser Ala Gly

<210>  6
<211>  19
<212>  PRT
<213>  Künstliche Sequenz

<220>
<221>  MISC_FEATURE
<223>  Beschreibung der künstlichen Sequenz: Peptid

<400>  6

Ser Leu Ser Ala Glu Phe Lys His Leu Thr Leu Arg Glu Gln Arg Cys
1               5                   10                  15

Gly Asn Gly

<210>  7
<211>  19
<212>  PRT
<213>  Künstliche Sequenz

<220>
<221>  MISC_FEATURE
<223>  Beschreibung der künstlichen Sequenz: Peptid

<400>  7

Gly Leu Ile Trp Asp Phe Gly Tyr Leu Thr Leu Val Glu Gln Arg Ser
1               5                   10                  15

Gly Gly Ser

<210> 8
<211> 17
<212> PRT
<213> Künstliche Sequenz

<220>
<221> MISC_FEATURE
<223> Beschreibung der künstlichen Sequenz: Peptid

<400> 8

Ser Leu Ala Ala Glu Phe Arg His Leu Gln Leu Lys Glu Gln Lys Asn
1               5                   10                  15

Ala

## Beschreibung

Die Erfindung betrifft die Verwendung von dimer-spezifischen Nuklear-Lokalisations-Signalpeptiden (dsNLS) abgeleitet aus der STAT DNA Bindungsdomäne. Diese Peptide können zur Diagnose und/oder Therapie von mit ihnen in Zusammenhang stehenden Krankheiten und als Targetmoleküle zum Screening von pharmakologischen Wirkstoffen eingesetzt werden. Außerdem betrifft die Erfindung die Peptide an sich und sie enthaltende therapeutische Mittel.

Bei vielen molekularen Prozessen zwischen Zellkern und Zytoplasma ist es erforderlich, dass ein gekoppelter Im- und Export von Komponenten zwischen Zellkern und Zytoplasma erfolgt. So wird z. B. die Signaltransduktion durch STAT-Proteine (Signal-Transducers and Activators of Transcription) so reguliert, dass sowohl Import als auch Export zwischen Zellkern und Zytoplasma in Regelkreisen reguliert werden. STAT-Proteine bilden eine hochkonservierte Gruppe von Transkriptionsfaktoren, die nach Stimulation durch Cytokine und Wachstumsfaktoren vielfältige Einflüsse auf die Genexpression ausüben. Die Aktivierung der STAT-Proteine erfolgt im Zytoplasma der Zelle an aktivierten Rezeptoren durch Phosphorylierung, worauf aktivierte STAT-Moleküle in den Kern transportiert werden, um dort die Transkription bestimmter Gene zu aktivieren oder zu reprimieren (Darnell, J.E., Jr. (1997) Science 277, 1630-1635). Die Cytokin-Signale gehen von Zelloberflächen-Rezeptoren aus. Die Bindung von Cytokinen oder Wachstumsfaktoren an ihre entsprechenden Rezeptoren initiiert eine Reihe von Tyrosin-Phosphorylierungen, welche durch Kinase-Mitglieder der Janus-Familie (Jaks) ausgelöst werden. Das führt zur Phosphorylierung einer Tyrosin-Seitenkette der STAT-Proteine. Dieser Prozess, gewöhnlich als STAT-Aktivierung bezeichnet, bewirkt die Dimerisierung der STAT-Proteine durch reziproke Phosphotyrosin/SH2-Interaktionen und später die schnelle und effiziente Translokation von cytoplasmischen Phospho-STAT-Molekülen in den Nuklearteil, wo sie spezifische Gene aktivieren oder reprimieren.

Die Cyotkin-induzierte Transkription ist ein vorübergehender Prozess, der wenige Minuten bis Stunden dauern kann. Zellen mit abweichend hoher und verlängerter Jak- oder STAT-Aktivierung sind anfällig für Transformationen und assoziiert mit abnormaler Entwicklung (Frank, D.A. (1999), Mol. Med. 5, 432-456). Details der dynamischen Umverteilung von Stat-Proteinen, die durch Cytokin-Aktivierung ausgelöst wird, sind zum größten Teil unbekannt.

Die Integration der im Kern und im Zytoplasma ablaufender Prozesse ist entscheidend bei Krebserkrankungen, bei Defekten des Immunsystems, bei Stoffwechselleistungen von Leber und Nieren und anderen.

Der Erfindung lag deshalb die Aufgabe zugrunde, Mittel und Verfahren bereitzustellen, die den Transport von Proteinen, Peptiden, Nucleinsäuresequenzen und anderen biologischen Strukturen, insbesondere von Transkriptionsfaktoren zwischen Zytoplasma und Zellkern modifizieren, sowie der Bereitstellung von Verfahren zum Screenen von Wirkstoffen, die sich zur Diagnose und Behandlung von mit Signalpeptiden, die den Transport zwischen Zellkern und Zytoplasma induzieren und regulieren, im Zusammenhang stehenden Krankheiten eignen.

Der Erfindung wird gemäß den Ansprüchen realisiert. Ihr liegt dabei die Erkenntnis zugrunde, dass ein nukleäres Importsignal existiert, welches spezifisch den Kerneintritt von Tyrosin-phosphoryliertem STAT-Dimer reguliert, und das dimer-spezifisches Nuklear-Lokalisations-Signal (dsNLS) genannt wird. Dieses Signal befindet sich in der DNA Bindungsdomäne. Die äußerst wichtigen identifizierten Reste sind innerhalb der STAT-Proteine gut konserviert, wobei dieses Signal von entscheidender Wichtigkeit für die Cytokin-aktivierbaren Funktionen dieser Transkriptionsfaktoren ist.

Das konservierte dsNLS ist entscheidend für den Interferony (IFN-γ) induzierten Kernimport von phosphorylierten STAT-Dimeren und benötigt zur Realisierung angrenzende positiv geladene und hydrophobe Reste. Weiterhin wurde ein konstitutives nukleocytoplastisches Pendeln von STAT zwischen Kern und Zytoplasma in Abwesenheit der IFN-γ Stimulierung festgestellt. Der Verlust des (ds) dimer-spezifischen Importsignals blockiert den Kerneintritt von Tyrosin-phosphoryliertem STAT, welches konsekutiv auch die Induktion von cytokin-induzierbaren Zielgenen verhindert. Der Kernimport von unphophoryliertem STAT wird durch das dsNLS nicht reguliert, so dass die STAT abhängige konstitutive Expression von Caspasen und die TNF-α vermittelte Apoptose-Induktion unverändert weiter geschehen. Folglich pendeln Tyrosin-phosphorylierte und unphosphorylierte STAT Moleküle über unabhängige Signalwege zu ihren jeweiligen Zielgenen.

Das bedeutet, dass der Transkriptionsfaktor STAT zwischen zwei unterschiedlichen nukleären Importwegen Phosphorylierungs-abhängig wechselt. Import als ein Tyrosin-phosphoryliertes Molekül erfordert eine intramolekulare Signalsequenz von Aminosäuren, die überraschend ein ungewöhnliches Kernimportsignal festlegt. Ein Verlust oder schwerwiegende Strukturveränderungen in diesem Signal beseitigt selektiv die Cytokin-induzierte Genaktivierung, während konstitutive transkriptionale Funktionen von STAT unverändert erhalten bleiben. Weiterhin wurde ein konstitutives und Tyrosin-Phosphorylierungsunabhängiges nucleocytoplasmisches Pendeln von STAT festgestellt. STAT ist also ein konstitutives Shuttle-Protein zwischen Zytoplasma und Kern vor der Cytokin-Stimulation.

Zusammenfassend ist festzustellen, dass eine Kernpräsenz von unphosphoryliertem STAT nicht die Vollständigkeit des Nuklear-Lokalisations-Signals (NLS) in der DNA Bindungsdomäne benötigt. Deshalb wurde der Terminus dimer-spezifisches (ds) NLS verwendet, um die Tatsache zu betonen, dass dieses Signal nur relevant für den Kernimport von Tyrosin-phosphorylierten STAT-Dimeren ist. Die Inaktivierung des dimer-spezifischen Nuklear-Lokalisations-Signals (dsNLS) blockiert selektiv die Cytokin-induzierte Geninduktion.

Das dsNLS in der DNA Bindungsdomäne ist notwendig für den Kernimport und die nachfolgende transkriptionale Aktivität von Tyrosin-phosphoryliertem STAT. Andererseits wird dieses Signal für konstitutive Funktionen von STAT-Monomeren nicht benötigt.

Tyrosin-phosphorylierte und unphosphorylierte STAT verwenden also eigene Mechanismen um in den Kern zu gelangen. Das STAT-dsNLS unterscheidet sich von konventionellen nukleären Lokalisationssignalen auf verschiedene Weise. Die am besten charakterisierten Beispiele von bekannten NLS Sequenzen sind durch Einzel- oder Doppel-Cluster von Basisresten gekennzeichnet (oft Lysin). Die STAT-dsNLS ähneln nicht diesem Konsens, doch sind positiv geladene Reste notwendig. Am Beispiel von STAT1 wurde festgestellt, dass zwei Lysine in den Positionen 410 und 413 für den Kernimport von Tyrosin-phosphorylierten Dimeren entscheidend sind. Jedoch wurde ein ausgedehntes Cluster von positiven Ladungen in dsNLS nicht gefunden und benachbarte Basis-Reste in den Positionen 405 und 406 beteiligten sich nicht an der Bildung des NLS.

Hydrophobe Reste sind demzufolge wichtig für die nukleäre Translokation von phosphoryliertem STAT. Während nämlich konservative Mutationen (Leucin, Isoleucin oder Valin), welche die hydrophobe Natur von Seitenketten konservieren, ohne Effekt auf einen Import waren, schloss die Einführung von Alanin oder Threonin in den Positionen 407 und 409 einen Kerneintritt von phosphoryliertem STAT1 aus. Mutationen von Aminosäure-Resten in den Positionen 407 und 409 der vollständigen STAT1-Moleküle von Leucin in Alanin oder Threonin beeinträchtigte signifikant die subzelluläre Verteilung. Diese Mutanten zeigten nach Cystein-Stimulation keine Translokation in den Zellkern. Diese Daten zeigen nachhaltig, dass die Mutante Leucin^{407,409} zu Alanin als ein Tyrosin phosphoryliertes Molekül nicht in den Nukleus transloziert werden kann.

Die Tyrosin-Phosphorylierung bestimmt demzufolge die Wahl des Signalweges im nukleocytoplastischen Pendeln von STAT. Dies erlaubt die schnelle und unabhängige Modulation zwischen Cytokin-induciblen und konstitutiven Funktionen von STAT.

Die Aufgabe der vorliegenden Erfindung wird durch Peptide gelöst, die eine Aktivität aufweisen, die den Transport von Proteinen, Peptiden, Nucleinsäuresequenzen, insbesondere Transkriptionsfaktoren zwischen Kern und Zytoplasma modifizieren.

Gegenstand der Erfindung ist deshalb die Verwendung von dimer-spezifischen Nuklear-Lokalisations-Signalpeptiden (dsNLS) abgeleitet aus der STAT DNA Bindungsdomäne, die dadurch gekennzeichnet sind, dass sie im Kontext von STAT-Molekülen bei der Transkription von Zielgenen zum Kernimport der STAT-Moleküle in Antwort einer Cytokin-Stimulation der Zellen geeignet sind und transkriptional regulatorische Eigenschaften aufweisen.

Insbesondere betrifft die Erfindung die Peptide an sich und ihre Verwendung und sie umfassender Fusionsproteine als Targetmoleküle zum Screening von Wirkstoffen, welche die Kernimport-Aktivität modifizieren. Unter Modifizieren wird dabei jede Beeinflussung der Kernimport-Aktivität verstanden, die zu einer Steigerung oder Verminderung der Aktivität führt, d.h. welche die Kernimport-Aktivität hemmen oder aktivieren.

In einer anderen Ausführungsvariante betrifft die Erfindung ihre Verwendung zur Diagnose und/oder Therapie von mit ihnen (den dsNLS) in Zusammenhang stehenden Krankheiten, insbesondere für die Diagnose und Therapie von Entzündungs- und Tumorerkrankungen sowie zur Detektion von Inhibitoren und Aktivatoren des Kernimports.

Diese Aktivitäten können mit an sich bekannten biologischen, chemischen oder physikalischen Meßmethoden bestimmt werden, wobei unter anderem
- die Akkumulation im Kern gemessen werden kann,
- die Exportaktivität aus dem Kern heraus oder auch
- die Aktivität eines Reportergens.

Deshalb sind Gegenstand der Erfindung die Verwendung der nativen Signalpeptide abgeleitet von unterschiedlichen STAT-Molekülen sowie die nativen Peptide an sich und ebenfalls die durch multiple Synthese darstellbaren Peptide. Die Peptide umfassen oder bestehen aus den folgenden bevorzugten Aminosäuresequenzen:
LLHELTIQSIQLELYHEELQLL; DdStat (546-567)
HVFSASFR--NMQLKKIK; DStat (426-441)
NCCSALFK--NLLLKKIK; hStat6 (354-369)
TL-SAHFR--NMSLKRIK; hStat5a (411-425)
SL-SVEFR--HLQPKEMKSSAG; hStat4 (394-412)
SL-SAEFK--HLTLREQRCGNG; hStat3 (403-421)
GL-IWDF--GYLTLVEQRSGGS; hStat2 (395-413)
SL-AAEFR--HLQLKEQKNA hStat1 (399-415).

Die Erfindung umfasst auch die Peptide, die biologisch aktive Varianten, Mutanten, oder Teile (Fragmente) von den obigen Sequenzen darstellen und/oder zu 80% homologe Sequenzen, wobei sie die gleichen Eigenschaften aufweisen. Gegenstand der Erfindung sind weiterhin die Peptide mit den obigen Sequenzen, in denen die Aminosäuren Leucin (L), Isoleucin (I) und/oder Valin (V) gegeneinander ausgetauscht sind. Gegeneinander ausgetauscht bedeutet im Sinne der Erfindung, dass L durch I oder V, I durch L oder V und V durch L oder I ausgetauscht sein kann, wobei die biologischen Aktivitäten der Peptide erhalten bleiben. Eingeschlossen sind auch Peptide in denen gegebenenfalls zusätzlich Arginin (R) und Lysin (K) gegeneinander ausgetauscht ist, ohne dass ihre biologische Aktivität verloren geht.

Die erfindungsgemäßen Peptide sind entweder gentechnisch durch rekombinante Nukleinsäuren oder durch multiple Synthese darstellbar. Ihre Herstellung erfolgt nach dem Fachmann bekannten biosynthetischen (mit Hilfe von Mikroorganismen) oder chemischen Techniken. So z.B. als ribosomale Peptid-Synthese (Translation), wobei eine Nukleotid-Sequenz von einem mRNA-Molekül abgelesen und in eine Sequenz von Aminosäuren übersetzt wird (Eur. J. Biochem. 236, 335-351 (1996). Die Erfindung betrifft auch eine Nucleinsäuresequenz, die ein Peptid oder ein Fusionsprotein codiert, oder einen komplementären Strang davon. Der Begriff Nucleinsäure betrifft ein natürliches oder synthetisches Polymer von DNA oder RNA, die einzel- oder doppelsträngig sein kann und in einer anderen Ausführungsform eine synthetische, nicht natürliche oder veränderte Nucleotidbase enthalten kann, die in DNA- oder RNA-Polymere eingebaut werden kann. Das Nucleinsäuremolekül kann cDNA, genomische DNA oder RNA, z. B. mRNA, sein. Die vorliegende Erfindung betrifft auch einen Vektor, der die vorstehend beschriebene Nucleinsäuresequenz umfaßt, insbesondere einen bakteriellen Vektor, wie ein Plasmid oder ein Virus. Überdies betrifft die vorliegende Erfindung Wirtszellen, die mit einem entsprechenden Vektor transformiert sind, insbesondere prokariotische oder eukariotische Zellen. Die vorliegende Erfindung betrifft auch Zellkulturen, Gewebe, Organe und dergleichen, die eine Zelle umfassen, die ein vorstehend beschriebenes Plasmid oder einen vorstehend beschriebenen Vektor enthalten.

Alternativ können Peptidsynthesen angewandt werden, wie sie z.B. in J.M. Stewart und J.D. Young "Solid Phase Peptide Synthesis", 2nd ed., Pierce Chemical Co., Rockford, Illinois (1984) und J. Meienhofer "Hormonal Proteins and Peptides", Vol. 2, Academic Press, New York (1973) für die Festphasensynthese und E. Schroder und K. Lubke "The Peptides", Vol. 1, Academic Press, New York, (1965) für die Flüssigsynthese beschrieben worden sind.

Ebenfalls Gegenstand der Erfindung sind Fusionsproteine, die die Peptide umfassen. Ihre herstellung ist dem Fachmann bekannt. Die verwendeten Fusionsproteine weisen mindestens ein Signalpeptid und mindestens eine Marker-Struktur auf. Marker-Strukturen und Reportergene sind an sich bekannt. Bevorzugt wird als Markerstruktur ein Green Fluorescent Protein-GFP, die Glutathion-S-Transferase und/oder ein Fragment hiervon verwendet, aber auch andere Fluoreszenzproteine, wie das rote oder gelbe, sowie das Maltose-bindende Protein (MBP) und weitere kommen in Frage. Das Green Fluorescent Protein weist vorteilhafterweise eine geringe oder überhaupt keine cytotoxische Aktivität auf. Mit Vorteil sind die Absorptions- und Emissionsmaxima des Green Fluorescent Protein ähnlich denen von Fluorescin, so daß beispielsweise eine GFP-positive Zelle mit gleichen Methoden nachgewiesen werden kann, wie solche Zellen, die mit Fluorescin-gekoppelten Antikörpern gefärbt wurden, das heißt beispielsweise im UV-Licht, unter dem Fluoreszenz-Mikroskop oder im Zellsorter (FACS).

Desweiteren betrifft die Erfindung den Einsatz der Signalpeptide und Fusionsproteine in Funktionstests, wie z.B. zur Identifizierung von spezifischen Antikörpern, insbesondere gegen die dsNLS sowie auch die Verwendung von Antikörpern, die gegen diese bevorzugten Peptid-Sequenzen und Fusionsproteine gerichtet sind.

Gegenstand der Erfindung sind darüber hinaus therapeutische Mittel, die die nativen oder synthetisch dargestellten Peptide oder sie umfassende Fusionsproteine in Applikationsformen für gentherapeutische Anwendungen aufweisen, z.B. in Form von Expressionsvektoren, zur Therapie von mit Kernimport-Signalpeptiden in Zusammenhang stehenden Krankheiten, wobei die Mittel den Kernimprot von endogenen Proteinen hemmen oder aktivieren können.

Krankheiten, die mit Kernimport-Signalpeptiden in Zusammenhang stehen, sind z.B. entzündliche Erkrankungen und Tumorerkrankungen, wie Arthritis, Carcinogenese, Nephritis, Proteinurie, Dermatitis, Diabetes/Fettleibigkeit, akute und chronische Abstoßung allogener Organtransplantate, Nervenzell-Degeneration, Parkinson-Krankheit, septischer Schock, Endotoxinämie, Hypersensibilität, Uveitis, Wundverschluß/Zellwachstum und andere. Die dsNLS spielen auch bei der Regulation des Immunsystems, Entzündungs- und Effektor-Mechanismen der zellulären und humoralen Immunität, septischem Schock, Entzündungen der Sinnesorgane, z.B. der Augen, bei der Regulation von Transkriptions- und Zellzyklen und akuter respiratorischer Insuffizienz, physiologischem Streß und anderen Störungen eine wichtige Rolle.

## Patentansprüche

1. Verwendung von dimer-spezifischen Nuklear-Lokalisations-Signalpeptiden (dsNLS) abgeleitet aus der STAT DNA Bindungsdomäne, die im Kontext von STAT-Molekülen bei der Transkription von Zielgenen zum Kernimport der STAT-Moleküle in Antwort einer Cytokin-Stimulation der Zellen geeignet sind und die transkriptional regulatorische Eigenschaften aufweisen sowie sie umfassende Fusionsproteine zur Herstellung eines Mittels
- zur Diagnose und/oder Therapie von mit dsNLS in Zusammenhang stehenden Krankheiten sowie
- als Targetmoleküle zum Screening von pharmakologischen Wirkstoffen, welche die Kernimport-Aktivität modifizieren, wobei die Peptide und Fusionsproteine aus der Aminosäuresequenz SL-SAEFK- -HLTLREQRCGNG bestehen.

2. Verwendung nach Anspruch 1,
**dadurch gekennzeichnet, dass** Peptide oder sie umfassende Fusionsproteine verwendet werden, in denen Leucin (L), Isoleucin (I) und/oder Valin (V) gegeneinander ausgetauscht sind sowie gegebenenfalls zusätzlich Arginin (R) mit Lysin (K) ausgetauscht ist.

3. Verwendung nach einem der Ansprüche 1 oder 2,
**dadurch gekennzeichnet, dass** das Fusionsprotein das Peptid in Verbindung mit mindestens einer Markerstruktur umfasst.

4. Verwendung nach Anspruch 3,
**dadurch gekennzeichnet, dass** die Markerstruktur ein green fluorescent protein (GFP), die Glutathion-S-Transferase und/oder ein Fragment hiervon ist und sich am NH-Ende und/oder COOH-Ende befindet.

5. Verwendung nach einem der Ansprüche 1 bis 4,
**dadurch gekennzeichnet, dass** gegen diese Peptide oder Fusionsproteine gerichtete Antikörper verwendet werden.

6. Verwendung nach einem der Ansprüche 1 bis 4,
**dadurch gekennzeichnet, dass** die Peptide und/oder Fusionsproteine zur Diagnose und/oder Therapie von Entzündungs- und Tumorerkrankungen verwendet werden, vorzugsweise Arthritis, in der Carcinogenese, Nephritis, Proteinurie, Dermatitis, Diabetes/Fettleibigkeit, akute und chronische Abstoßung allogener Organtransplantate, Nervenzell-Degeneration, Parkinson-Krankheit, septischer Schock, Endotoxinämie, Hypersensibilität, Uveitis, Wundverschluß/Zellwachstum, zur Regulation des Immunsystems, bei Entzündungs- und Effektor-Mechanismen der zellulären und humoralen Immunität, septischem Schock, Entzündungen der Sinnesorgane, zur Regulation von Transkriptions- und Zellzyklen und akuter respiratorischer Insuffizienz und/oder physiologischem Streß.

7. Verwendung nach einem der Ansprüche 1 bis 6,
**dadurch gekennzeichnet, dass** die Diagnose anhand von Gewebeproben aus dem Zytoplasma unter Verwendung entsprechender Antikörper erfolgt.

8. Peptide abgeleitet aus der STAT DNA Bindungsdomäne, die im Kontext von STAT-Molekülen bei der Transkription von Zielgenen zum Kernimport der STAT-Moleküle in Antwort einer Cytokin-Stimulation der Zellen geeignet sind und die transkriptional regulatorische Eigenschaften aufweisen sowie sie umfassende Fusionsproteine, wobei die Peptide, aus der Aminosäuresequenz SLSAEFKHLTLREQRCGNG bestehen.

9. Peptide nach Anspruch 8 in denen Leucin (L), Isoleucin (I) und/oder Valin (V) gegeneinander ausgetauscht sind sowie Peptide, in denen zusätzlich Arginin (R) und Lysin (K) gegeneinander ausgetauscht ist, wobei ihre biologische Aktivität erhalten bleibt.

10. Nucleinsäuresequenz, die ein Peptid nach einem der Ansprüche 8 oder 9 oder ein sie umfassendes Fusionsprotein oder einen komplementären Strang davon codiert.

11. Vektor, umfassend die Nucleinsäuresequenz nach Anspruch 10.

12. Wirtszelle, umfassend den Vektor nach Anspruch 11.

13. Therapeutische Mittel zur Behandlung von entzündlichen oder Tumorerkrankungen umfassend native dimer-spezifische Nuklear Lokalisations-Signalpeptide (dsNLS), aus der Aminosäuresequenz SLSAEFKHLTLREQRCGNG bestehend, die zur Expression kommen und den Kernimport von endogenen nativen Proteinen beeinflussen.

14. Mittel nach Anspruch 13,
**dadurch gekennzeichnet, dass** sie in Formulierungen zur gentherapeutischen Anwendung vorliegen, z.B. in Form von Expressionsvektoren.
